# EUROPEAN PATENT SPECIFICATION

(11) **EP 1 713 937 B1**
(45) Date of publication and mention of the grant of the patent: **17.08.2016**
(21) Application number: 05716622.5
(22) Date of filing: 03.02.2005
(51) Int. Cl.: C12Q 1/68

(54) **DETECTION, IDENTIFICATION AND DIFFERENTIATION OF PROTEUS SPECIES USING THE SPACER REGION**
NACHWEIS, IDENTIFIZIERUNG UND DIFFERENZIERUNG VON PROTEUS SPEZIES UNTER VERWENDUNG DER REGION ZWISCHEN DEN 16S UND 23S RNA GENEN
DETECTION, IDENTIFICATION ET DIFFERENCIATION DES ESPECES PROTEUS A PARTIR DE LA REGION DES ESPACEURS

(30) Priority: 06.02.2004 EP 04447030; 10.02.2004 US 542875 P
(43) Date of publication of application: 25.10.2006
(73) Proprietor: Fujirebio Europe N.V., 9052 Gent (BE); Roche GmbH, 68305 Mannheim (DE)
(72) Inventor: JANNES, Geert, B-3010 Leuven (BE); MIJS, Wouter, B-9820 Merelbeke (BE); HABERHAUSEN, Gerd, 82377 Penzberg (DE); EMRICH, Thomas, 82393 Iffeldorf (DE)
(74) Representative: De Clercq, Ann G. Y.
(86) International application number: PCT/EP2005/050464
(87) International publication number: WO 2005/075673

(56) References cited:
- WO-A-00/52203
- WO-A-00/66785
- WO-A-03/095677
- WO-A1-96/00298
- US-A- 6 025 132
- US-B1- 6 512 105

## Description

### FIELD OF THE INVENTION

The present invention relates to new nucleic acid sequences derived from the ITS (Internal Transcribed Spacer) region, between the 16S and 23S ribosomal ribonucleic acid (rRNA) or rRNA genes, to be used for the specific detection and/or identification of *Proteus* species

The present invention relates also to a method for the specific detection and/or identification of *Proteus* species, using new nucleic acid sequences derived from the ITS region.

Said sequence or method may be used to detect and/or identify *Proteus mirabilis, Proteus vulgaris,* and/or *Proteus penneri.*

### BACKGROUND OF THE INVENTION

The genus *Proteus* consists of 8 species: *P. mirabilis, P. penneri, P. vulgaris, P. myxofaciens* and *P. hauseri and* 3 genomospecies not yet named.

Members of the genus *Proteus,* are commonly found in the environment while they often also make up part of the gastrointestinal tract. Clinically, *P. mirabilis* is the most relevant as most frequently isolated organism although the other species can be encountered too in the clinical setting.

*P. mirabilis* accounts for 3% of isolates from nosocomial infections while it ranks second, after *Escherichia coli,* among isolates of common urinary tract infections and third as causative agent of uncomplicated cystitis, pyelonephritis and *prostatitis. P. mirabilis* is also reported as etiologic agent of those life-threatening infections such as bacteremia, neonatal meningo-encephalitis, meningitis, empyema and osteomyelitis. Also, other infections such as gastrointestinal and wound infections could be caused by *P. mirabilis* and related species such as *P. penneri.*

*P. penneri,* as well as *P. mirabilis,* were shown to be implicated in kidney stone formation, while *P. mirabilis* has been reported as an etiopathologic agent in rheumatoid arthritis.

Currently the *Proteus* species are identified and differentiated by culture based methods and phenotypic biochemical tests.

A typical characteristic for *Proteus* is the swarming property of the bacterium on sheep blood agar. In combination with an oxidase and indol test the different *Proteus* species can be differentiated with accuracy although not all the cases can be resolved in a clear cut way by the traditional systems. Current, commercially available systems do not give a uniform and unique answer in the identification of and the differentiation between *Proteus* species.

Besides their inherent resistance to nitrofurantoin and tetracycline most of those *Proteus* spp. are, as wild-type strains, susceptible to amino/ureido penicillins, cephalosporins, aminoglycosides and carbapenems. However, recent reports show the emergence of resistances against several antimicrobial agents amongst others against the mentioned ones, particularly in some hospitals. A rapid and specific identification assay for those organisms could form the basis for a more appropriate antimicrobial management of infections caused by these typical opportunistic bacterial organisms.

Taking into account the increasing number of nosocomial infections as well as the increase in resistance to the existing panel of antimicrobial agents, and since culture based testing is still time consuming and requiring a high workload from skilled personnel, new methods for rapid and more specific identification are needed. In particular in the case of serious infections, like nosocomial sepsis, a rapid, specific and sensitive assay is mandatory because it is a question of life or death.

The international patent application WO 03/095677 describes a few probes from the badly characterized 23S and ITS rRNA genes *of P. vulgaris* for identifying this specific species, describing by accident probe ATACGTGTTATGTGC from the ITS region.

A method for identifying bacteria in a sample with the *P. mirabilis* species from the *Proteus* group only present, by amplifying a portion of the 23S rDNA present in the sample has been disclosed in the international patent application WO 00/52203.

There is however a need for a method to identify not only whether a *Proteus* species is present in a sample but also which type of *Proteusspecies* is present.

### SUMMARY OF THE INVENTION

It is an object of the present invention to provide new nucleic acid sequences derived from the ITS of *Proteus* species, which can be used, for the detection and/or identification of *Proteus* species. Sequences derived from the ITS *Proteins* species, in particular of *Proteus mirabilis, Proteus vulgaris,* and/or *Proteus penneri* are provided.

The present invention provides subject-matter as set forth in any one and all of (i) to (viii) below:
(i) A set of at least two polynucleotide probes for the detection and/or identification of Proteus species, each probe comprising 5 to 50 nucleotides and said probes hybridizing specifically at adjacent positions separated by no more than 25 nucleotides between said two probes in any of nucleic acids selected from the group consisting of SEQ ID NO 1to17, their RNA form wherein T is replaced by U, the complementary form thereof or equivalent homologous polynucleotides sharing at least 80% homology with the corresponding unmodified polynucleotides of SEQ ID NO 1 to 17.
(ii) A set of at least two polynucleotide probes as set forth in (i) above, wherein said probes are selected from the group consisting of SEO ID NO 18 to 67.
(iii) A set of three polynucleotide probes as set forth in (i) or (ii) above, each probe comprising 5 to 50 nucleotides and said probes hybridizing specifically at adjacent positions separated by no more than 25 nucleotides between adjacent probes in any of nucleic acids selected from the group consisting of SEQ ID NO 1to17, their RNA form wherein T is replaced by U, the complementary form thereof, or equivalent homologous polynucleotides sharing at least 80% homology with the corresponding unmodified polynucleotides of SEQ ID NO 1to17.
(iv) A composition comprising at least one set of at least two polynucleotide probes as set forth in any one of (i) to (iii) above.
(v) A method for detecting or identifying Proteus species using at least one set of at least two polynucleotide probes as set forth in any one of (i) to (iii) above, wherein said probes are selected from the group consisting of SEO ID NO 18 to 67.
(vi) A method as set forth in (v) above for detection and/or identification of Proteus species in a sample comprising the steps of:
   (i) releasing, isolating and/or concentrating the polynucleic acids in the sample;
   (ii) amplifying the 16S-23S rRNA spacer region(s), or at least one of the target sequence(s) which comprise(s) any nucleic acid molecule(s) as set forth in (i) above, with at least one suitable primer pair;
   (iii) contacting the polynucleic acids with at least one set of at least two polynucleotide probes hybridizing specifically at adjacent positions separated by no more than 25 nucleotides between adjacent probes, said probes selected from the group consisting of SEQ ID NO 18 to 67;
   (iv) detecting the hybrids formed, and
   (v) interpreting the signal(s) obtained and inferring the presence of Proteus species and/or identifying the Proteus species in the sample.
(vii) A method as set forth in (v) or (vi) above wherein the two polynucleotide probes consist of any combination of polynucleotides of Table 2.
(viii) A kit for detection and/or identification of Proteus species comprising the following components:
   - a set of at least two polynucleotide probes as set forth in any one of (i) to (iii) above,
   - a hybridization buffer, or components necessary for producing said buffer.

The present specification describes an isolated nucleic acid molecule selected from the group consisting of SEQ ID NOs 1 to 67, their complementary form, the RNA form thereof wherein T is replaced by U, and homologues.

According to the present specification, said nucleic acid molecules may be used for the detection and/or identification of *Proteus* species.

The present specification describes new polynucleotides for use as probes and/or primers, for the detection and/or identification of *Proteus* species, in particular of *Proteus mirabilis, Proteus vulgaris,* and/or *Proteus penneri.*

The present specification thus describes an isolated nucleic acid molecule that specifically hybridizes to a target sequence comprising or consisting of a nucleic acid molecule selected from the group consisting of SEQ ID NOs 18 to 67, their complementary form, the RNA form thereof wherein T is replaced by U, homologous sequences thereof, and fragments thereof, for the detection and/or identification of *Proteus* species.

The present specification further describes sets of probes for the detection and/or identification of *Proteus* species, in particular of *Proteus mirabilis, Proteus vulgaris,* and/or *Proteus penneri* in a sample.

The present specification also describes primers allowing specific amplification of the 16S-23S rRNA spacer region of *Proteus* species, in particular of *Proteus mirabilis, Proteus vulgaris,* and/or *Proteus penneri.*

The present specification further describes a composition containing any of the new sequences described herein, or any of the new sets of probes and/or primers described herein, or a combination thereof.

The present specification further describes a kit, in which said probes and/or primers are used, for the detection and/or identification of *Proteus* species, in particular of *Proteus mirabilis, Proteus vulgaris,* and/or *Proteus penneri.*

The present specification also describes a rapid and reliable hybridization method for detection and/or identification of *Proteus* species, in particular of *Proteus mirabilis, Proteus vulgaris,* and/or *Proteus penneri.*

The present specification further describes a hybridization method based on real time PCR for detection and/or identification of *Proteus* species, in particular *Proteus mirabilis, Proteus vulgaris,* and/or *Proteus penneri.*

### TABLE LEGENDS

Table 1: Amplification and melting curve program used in the examples.
Table 2: Different combinations of HybProbes tested
Table 3: list of microorganisms tested for specificity of the combination of HybProbes represented by SEQ ID NO 24 and 39.
Table 4: list of SEQ ID NOs 1 to 69.
The SEQ ID's from this table are derived from the following organisms:

| **Seq ID** | **Organism** | **Seq ID** | **Organism** |
|---|---|---|---|
| 1 | *P. mirabilis (glu)* | 25 | *PROTEUS* |
| 2 | *P. mirabilis* (*glu*) | 26 | *PROTEUS* |
| 3 | *P. mirabilis (glu)* | 27 | *P. mirabilis (glu)* |
| 4 | *P. mirabilis (flu)* | 28 | *P. mirabilis (glu)* |
| 5 | *P. mirabilis (ile-ala)* | 29 | *P. mirabilis (glu)* |
| 6 | *P. mirabilis (ile-ala)* | 30 | *PROTEUS* |
| 7 | *P. mirabilis (ile-ala)* | 31 | *PROTEUS* |
| 8 | *P. mirabilis (ile-ala)* | 32 | *PROTEUS* |
| 9 | *P. mirabilis (ile-ala)* | 33 | *PROTEUS* |
| 10 | *P. mirabilis (ile-ala)* | 34 | *P. mirabilis (ile-ala)* |
| 11 | *P. vulgaris* | 35 | *P. mirabilis (ile-ala)* |
| 12 | *P. vulgaris* | 36 | *PROTEUS* |
| 13 | *P. vulgaris* | 37 | *P. mirabilis (glu)* |
| 14 | *P. penneri* | 38 | *P. mirabilis (glu)* |
| 15 | *P. penneri* | 39 | *P. mirabilis (glu)* |
| 16 | *P. penneri* | 40 | *PROTEUS* |
| 17 | *P. penneri* | 41 | *PROTEUS* |
| 18 | *P. vulgaris (glu)* | 42 | *PROTEUS* |
| 19 | *P. vulgaris (glu* + *ile*/*ala)* | 43 | *PROTEUS* |
| 20 | *P. vulgaris (glu* + *ile*/*ala)* | 44 | *PROTEUS* |
| 21 | *P. mirabilis (glu)* | 45 | *PROTEUS* |
| 22 | *P. mirabilis (glu)* | 46 | *PROTEUS* |
| 23 | *P. mirabilis (glu)* | 47 | *PROTEUS* |
| 24 | *P. mirabilis (glu)* | 48 | *P. mirabilis* |
| 49 | *PROTEUS* | 60 | *P. vulgaris* |
| 50 | *P. vulgaris* + *P. penneri (glu)* | 61 | *PROTEUS* |
| 51 | *P. mirabilis* | 62 | *PROTEUS* |
| 52 | *P. vulgaris* + *P. penneri (glu)* | 63 | *PROTEUS* |
| 53 | *PROTEUS* | 64 | *PROTEUS* |
| 54 | *P. mirabilis* (*ile*/*ala*) | 65 | *P. mirabilis* |
| 55 | *P. mirabilis (ile*/*ala)* | 66 | *P. mirabilis* |
| 56 | *P. vulgaris* (*ile*/*ala*) | 67 | *P. mirabilis* |
| 57 | *P. vulgaris (ile*/*ala)* | 68 | *PRIMERS* |
| 58 | *PROTEUS* | 69 | *PRIMERS* |
| 59 | *PROTEUS* | | |

### DETAILED DESCRIPTION OF THE INVENTION

The following definitions serve to illustrate the terms and expressions used in the different embodiments of the present invention as set out below.

The terms "spacer" and "ITS" (Internal Transcribed Spacer) are abbreviated terms both referring to the region between the 16S and 23S rRNA or between the 16S and 23S rRNA genes.

The term "probe" refers to a single stranded oligonucleotide or a polynucleotide which has a sequence which is sufficiently complementary to hybridize to a target sequence.

A target sequence is a sequence to be detected comprising any nucleic acid molecule represented by any of the SEQ ID NOs 1 to 17, their complementary form, RNA form thereof, homologues or fragments thereof.

A target sequence can be either genomic DNA or precursor RNA, or amplified versions thereof.

Preferably the probes described herein are about 80%, about 85%, about 90%, or more than about 95% homologous to the exact complement of the target sequence.

The probes described herein can be formed by cloning (and growing) of recombinant plasmids containing inserts including the corresponding nucleotide sequences, if need be by cleaving the latter out from the cloned plasmids using the adequate nucleases and recovering them, e.g. by fractionation according to molecular weight.

The probes described herein can also be synthesized chemically, for instance by the conventional phospho-triester method.

The term "complementary" nucleic acids as used herein means that the nucleic acid sequences can form a perfect base-paired double strand with each other.

The terms "polynucleic acid", "nucleic acid", and "polynucleotide" correspond to either double-stranded or single-stranded cDNA or genomic DNA or RNA, containing at least 5, 10, 15, 20, 30, 40 or 50 contiguous nucleotides. A polynucleic acid, which is smaller than 100 nucleotides in length is also referred to as an "oligonucleotide".

The polynucleotides described herein can also contain modified nucleotides such as inosine or nucleotides containing modified groups which do not essentially alter their hybridization characteristics.

The polynucleic acid molecules described herein are always represented from the 5' end to the 3' end. They can be used in any form, i.e. their double-stranded or single-stranded form (any of the two strands), their DNA or RNA form (wherein T is replaced by U), modified or not.

The term "closest neighbour" means the taxon, which is known or expected to be the most closely related in terms of DNA homology and which has to be differentiated from the organism of interest.

The expression "taxon-specific hybridization" or "taxon-specific probe" means that the probe only hybridizes to the DNA or RNA from the taxon for which it was designed and not to DNA or RNA from other taxa.

The term taxon can refer to a complete genus or a sub-group within a genus, a species or even subtype within a species (subspecies, serovars, sequevars, biovars ... ).

The term "specific amplification" or "specific primers" refers to the fact that said primers only amplify the relevant region from the organisms for which they were designed, and not from other organisms.

The term "spacer specific amplification" or "spacer specific primers" refers to the fact that said primers only amplify the spacer region from the organisms for which they were designed, and not from other organisms.

The term "specific probe" refers to probes that only hybridize with the relevant region from the organisms for which they were designed, and not with the corresponding region from other organisms, nor with any other region.

The term "spacer specific probe" refers to probes that only hybridize with the relevant spacer from the organisms for which they were designed, and not with spacers from other organisms.

The term "sensitivity" refers to the number of false negatives: i.e. if 1 of the 100 strains to be detected is missed out, the test shows a sensitivity of (100-1/100)% = 99%.

The term "specificity" refers to the number of false positives: i.e. if on 100 strains
detected, 2 seem to belong to organisms for which the test is not designed, the specificity of the test is (100-2/100)% = 98%.

The oligonucleotides or polynucleotides selected as being "preferential" show a sensitivity and specificity of more than 80%, preferably more than 90% and most preferably more than 95%.

The term "solid support" can refer to any substrate to which a polynucleotide probe can be coupled, provided that the probe retains its hybridization characteristics and provided that the background level of hybridization remains low. Usually the solid substrate will be a microtiter plate, a membrane (e.g. nylon or nitrocellulose) or a microsphere (bead), without being limited to these examples. Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin, haptens or proteins.

The term "labeled" refers to the use of labeled nucleic acids. Labeling may be carried out by the use of labeled nucleotides incorporated during the polymerization step of the amplification such as illustrated by Saiki et al. ((1988) Science 239:487-491) or Bej et al. ((1990) Mol Cell Probes 4:353-365) or by the use of labeled primers, or by any other method known to the person skilled in the art. The nature of the label may be isotopic ³³P, ³⁵S, etc.) or non-isotopic (biotin, digoxigenin, fluorescent dye, enzyme, etc.).

The term "signal" refers to a series of electromagnetic waves (for example fluorescence), or changes in electrical current which carry information. The signal can be directly visible, or can be made visible and/or interpretable by different means or devices.

A sample may comprise any biological material. This biological material may be taken either directly from the infected human being, or animal, or after culturing or enrichment, or from food, from the environment, etc.

Biological material may be for example expectoration of any kind, broncheolavages, blood, skin tissue, biopsies, lymphocyte blood culture material, colonies, etc. Said samples may be prepared or extracted according to any of the techniques known in the art.

According to the present specification, the *Proteus* species that are clinically relevant may be *Proteus mirabilis, Proteus vulgaris* and *Proteus penneri.*

Different *Proteus* species show two different types of spacer based on the type of tRNA gene inserted in the spacer region, tRNA^{glu} or tRNA^{ile-ala}. Moreover, for each type of spacer and for each *Proteus species,* different clusters or groups can be distinguished.

For instance, out of nine strains *of P. mirabilis,* having regard to the first type of spacer, i.e. with insertion of tRNA^{glu}, four different groups could be defined, represented respectively by SEQ ID NOs 1 to 4.

Having regard to the second type, i.e. with insertion of tRNA^{ile-ala}, six different groups could be defined, represented respectively by SEQ ID NOs 5 to 10.

To detect and/or identify all *Proteus* species, or each *Proteus* species, or any combination of at least two *Proteus* species, the present invention provides a set of at least two new nucleic acid molecules.

An ITS sequence described herein comprises or consists of a nucleic acid molecule selected from the group consisting of SEQ ID NO 1 to 17, their complementary form, the RNA form thereof wherein T is replaced by U, and any homologous sequences thereof.

Homologous sequences found in the ITS of any *Proteus* species, also referred to herein after as "homologues", are also described. The degree of homology is higher than 80% or 85%, preferably higher than 90%, and more preferably higher than 95%.

"homologues" may be homologous sequences to any of SEQ ID NOs 1 to 17 or to any fragment thereof of at least 10, 15, 20, 25, 30, 40, 50, 60, 70, 80, 90, 100 nucleotides, localized in the ITS region of any *Proteus* species.

SEQ ID NOs 1 to 10 are derived from *P. mirabilis,* SEQ ID NOs 11 to 13 are derived from *P. vulgaris* and SEQ ID NOs 14 to 17 from *P. penneri.*

The present specification also describes new nucleic acid molecules derived from the ITS for the detection of any *Proteus* species, solving the problems generated by a very high variability due to the fact that there are different types of ITS having regard to the tRNA inserted, each type comprising different groups.

Indeed, it has been discovered that the new nucleic acid molecules consisting of SEQ ID NO 44, 53, 58, 59 and 61 are found in the two types of spacers of every *Proteus* species tested, notably the *Proteusspecies* that are clinically relevant.

The mentioned specific polynucleotides, any fragments thereof of at least 10, 15, 20, 25, 30, and preferably of about 20 nucleotides (18, 19, 20, 21, or 22), the RNA form thereof and the complementary form thereof, also referred as genus-specific polynucleotides, are specific regions of the ITS that can be used for designing primers and/or probes for the detection of any or all of the *Proteus* species, in particular of the three *Proteus* species that are clinically relevant.

New polynucleotides for use as probes and/or primers for the detection and/or identification of one, two or more *Proteus* species are also provided.

In other words, the present specification describes new polynucleotides for use as probes and/or primers, which hybridize with the target sequences described herein for the detection and/or identification of one, two or more *Proteus* species.

In particular, the present specification describes an isolated nucleic acid molecule that specifically hybridizes to a target sequence comprising or consisting of a nucleic acid selected from the group consisting of SEQ ID NO 1 to 17, their RNA form wherein T is replaced by U, the complementary form thereof, any homologues thereof, and fragments of at least 10, 15, 20, 25, 30, 50, 100, 150, 200, or 300 contiguous nucleotides thereof.

Preferred polynucleotide probes are between about 5 to about 50 bases in length, more preferably from about 10 to about 25 nucleotides and are sufficiently homologous to the target sequence.

Polynucleotides of SEQ IDs NO 18 to 67 or any of their homologues, the complementary form thereof or the RNA form thereof may be used as probes.

Preferred primers described herein are single stranded DNA polynucleotides capable of acting as a point of initiation for synthesis of the target sequence. The length and the sequence of a primer described herein must be such that they allow to prime the synthesis of the extension products.

Preferably a primer described herein is about 5 to about 50 nucleotides long, preferably about 10 to about 35, more preferably about 15 to about 25. Its specific length and sequence is to be chosen depending on the conditions used such as temperature and ionic strength.

Primers described herein amplify the target sequences. In other words, primers described herein amplify a nucleic acid molecule comprising any of SEQ ID NOs 1 to 17, their complementary strand and/or homologues.

Universal primers located in the conserved flanking regions of the rRNA spacer, i.e. in the 16S gene and the 23S gene, can be used. If *Proteus* species are present in the sample, the amplification product, the target sequence(s), will then comprise a nucleic acid molecule consisting of any of SEQ ID NOs 1 to 17 and/or homologues.

Preferably, the target sequence(s) consist(s) of any nucleic acid molecules selected from the group consisting of SEQ ID NOs 1 to 17 and/or homologues, flanked by no more than about 40 to about 50 nucleotides of respectively the 16S and 23S rRNA.

For some applications it may be appropriate to amplify not different bacteria present in the sample but more specifically *Proteus* species.

In this case a primer pair is derived from the ITS sequences described herein, for example from the polynucleotides represented by SEQ ID NO 44 and 53.

The fact that amplification primers do not have to match exactly with the corresponding template sequence to warrant proper amplification is amply documented in the literature (Kwok et al. (1990) Nucl Acids Res. 18:999).

The amplification method used can be either polymerase chain reaction (PCR; Saiki et al., ((1988), Science 239:487-491), ligase chain reaction (LCR; Landgren et al., ((1988), Science 241:1077-1080), Wu & Wallace, ((1989) Genomics 4:560-569); Barany, ((1991), Proc Natl Acad Sci. USA 88:189-193) nucleic acid sequence-based amplification (NASBA; Guatelli et al., ((1990), Proc Natl Acad Sci. USA 87:1874-1878), Compton, ((1991), Nature 350:91-92) transcription-based amplification system (TAS; Kwoh et al, (1989) Proc Natl Acad Sci. USA 86:1173-1177), strand displacement amplification (SDA; Duck, ((1990) Biotechniques 9:142-147); Walker et al., ((1992) Proc Natl Acad Sci. USA 89:392-396) or amplification by means of Qβ replicase (Lizardi et al., ((1988) Bio/Technology 6:1197-1202), Lomeli et al., ((1989) Clin Chem 35:1826-1831) or any other suitable method to amplify nucleic acid molecules known in the art.

The preferred polynucleotides described herein for use as primers or as probes, or for designing further primers and probes to be used in methods described herein, are represented by SEQ ID NOs 18 to 67.

Polynucleotides described herein may differ in sequence from any of the polynucleotides represented by SEQ ID NO 18 to 67, either by addition to or removal from any of their respective extremities of one or several nucleotides, or by changing one or more nucleotides within said sequences, or a combination of both, provided that the equivalents then obtained still hybridize with the target sequence. Said equivalent polynucleotides share at least 80% homology, preferably more than 85%, most preferably more than 90% homology with the corresponding unmodified polynucleotides.

When using an equivalent of a polynucleotide, it may be necessary to modify the hybridization conditions to obtain the same specificity as the corresponding unmodified polynucleotide.

As a consequence, it will also be necessary to modify accordingly the sequence of other polynucleotides when the polynucleotides are to be used in a set under the same hybridization conditions. These modifications can be done according to principles such as those described in Hames B and Higgins S (Eds): Nucleic acid hybridization. Practical approach. IRL Press, Oxford, UK, 1985.

The polynucleotides primers and/or probes described herein may also comprise nucleotide analogues such as phosphorothioates (Matsukura et al., ((1987) Proc Natl Acad Sci. USA 84(21):7706-7710), alkylphosphorothioates (Miller et al., ((1979), Biochemistry 18(23):5134-5143) or peptide nucleic acids (Nielsen et al., ((1991) Science 254(5037):1497-1500); Nielsen et al., ((1993) Nucl Acids Res. 21(2):197-200) or may contain intercalating agents (Asseline et al., (1984), ). Proc Natl Acad Sci. USA 81(11):3297-3301) etc.

The modified primers or probes require adaptations with respect to the conditions under which they are used in order to obtain the required specificity and sensitivity. However the results of hybridization should remain essentially the same as those obtained with the unmodified polynucleotides.

The introduction of these modifications may be advantageous in order to influence some characteristics such as hybridization kinetics, reversibility of the hybrid-formation, biological stability of the polynucleotide molecules, etc.

The probes and primers described herein are used in methods, for the detection and/or identification of *Proteus* species, in particular of *Proteus mirabilis, Proteus vulgaris,* and/or *Proteus penneri.*

Detection and/or identification of the target sequences can be performed by using an electrophoresis method, a hybridization method or a sequencing method.

According to the specification, a method for the detection of one or more *Proteus* species in a sample may comprise the following steps:
- First, and if necessary, the nucleic acids present in the sample are made available for amplification and/or hybridization.
- Secondly, and also if necessary, the nucleic acids, if present, are amplified with one or another target amplification system. Usually, amplification is needed to enhance the subsequent hybridization signal. However for some samples, or for some highly sensitive signal-amplification systems, amplification might not be necessary.
- Thirdly, the nucleic acids present in the sample or the resulting amplified product are contacted with probes, and hybridization is allowed to proceed.
- Finally, the hybrids are detected using a convenient and compatible detection system. From the hybridization signal(s) or pattern(s) observed the presence or absence of one, two or more *Proteusspecies* can be deduced.

For the amplification step, primers located in the conserved flanking regions (16S and 23S gene) of the rRNA spacer, also called universal primers, can be used. The primer pair represented by SEQ ID NOs 68 and 69 is an example of a universal primer pair.

For some applications it may be appropriate to amplify not all bacteria present in the sample but one or several genera, or one or several *Proteus* species.

In the latter case, this may be achieved by using genus specific primers or species specific primers derived from the ITS region of *Proteus* species.

According to the specification" a method for detection and/or identification of *Proteus* species in a sample may comprise the steps of:
(i) optionally, isolating and/or concentrating the polynucleic acids present in the sample;
(ii) optionally amplifying the 168-238 rRNA spacer region(s), or at least one of the target sequences or (a) fragment(s) thereof, with at least one suitable primer pair;
(iii) contacting the polynucleic acids with at least one polynucleotide probe that hybridizes to at least one of the target sequences selected from the group consisting of SEQ ID NOs 1 to 17, homologues thereof, their RNA form wherein T is replaced by U, the complementary form thereof and fragments thereof;
(iv) detecting the hybrids formed, and
(v) interpreting the signal(s) obtained and inferring the presence of *Proteus* species and/or identifying the *Proteus* species in the sample.

A fragment, as mentioned for instance in the amplification or the hybridization step of any method described herein, may comprise or consist of about 10, 15, 20, 25, 30, 50, 100, 200, 300 contiguous nucleotides of a nucleic acid molecule described herein.

Preferably, the probes described hereinhybridize under conditions of high stringency.

Under high stringency conditions only complementary nucleic acid hybrids are formed. Accordingly, the stringency of the assay conditions determines the amount of complementarity needed between two nucleic acid strands forming a hybrid. Stringency is chosen to maximize the difference in stability between the hybrid formed with the target and the non-target nucleic acid.

In any case, the appropriate hybridization conditions are chosen in such a way that the signal of hybridization obtained when a polynucleotide described hereinhybridizes specifically to a target sequence, is different from the signal obtained when said polynucleotide hybridizes to a target sequence in a non-specific manner.

In practice, the different signals may be visualized for example when its intensity is two, five, ten or more times stronger with a specific hybridization to the target, as compared to non-specific hybridization to the target sequence. The LiPA system is a good example in this respect.

The different signals may also be visualized when different peaks are drawn in a melting curve analysis, for instance when using a real time PCR method.

In one embodiment, a very convenient and advantageous technique for the detection of target sequences that are possibly present in the sample is the real time PCR method.

There are different formats for the detection of amplified DNA that can be used, notably TaqMan™ probes, Molecular Beacons probes, "Scorpions", or FRET hybridization probes.

Concerning the TaqMan™ probes, a single-stranded hybridization probe is labelled with two components. When the first component, the so-called fluorescer, is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the polymerase, for example Taq Polymerase, during the elongation phase. As a result the excited fluorescent component and the quencher are spatially separated from one another and thus a fluorescence emission of the first component can be measured (EP patent 543 942 and US patent 5,210,015).

Concerning Molecular Beacons probes, the probes are also labeled with a first component and with a quencher, the labels preferably being located at different ends of an at least partially self-complementary probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US patent 5,118,801).

Concerning "Scorpions", a probe and a primer are contained in one molecule. Similarly to the Molecular Beacons system, each probe is labeled with a first component and with a quencher, the labels being located at different ends of an at least partially selfcomplementary probe. A primer is linked to each probe by the intermediary of a PCR stopper, which prevents the secondary structure from being opened in the absence of the specific target sequence. (Whitcombe, D. et al. (1999) Nature Biotechnology 17, 804-807; Thelwell, N. et al. (2000) Nucleic Acids Research vol. 28, No 19, 3752-3761; Svanvik et al Analytical Biochemistry 287, 179-182 (2000).

The Fluorescence Resonance Energy Transfer (FRET) hybridization probe test format is especially useful for all kinds of homogenous hybridization assays (Matthews, J. A. and Kricka, L. J., Anal Biochem 169 (1988) 1-25). It is characterized by two single-stranded hybridization probes which are used simultaneously and are complementary to adjacent sites of the same strand of an (amplified) target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured only when both hybridization probes bind to adjacent positions of the target molecule to be detected.

When annealed to the target sequence, the hybridization probes must be located very close to each other, in a head to tail arrangement. Usually, the gap between the labeled 3' end of the first probe and the labeled 5' end or the second probe is as small as possible, and notably consists of about 0 to 25 bases, and preferably of about 1 to about 5 bases. This allows for a close vicinity of the FRET donor compound and the FRET acceptor compound, which is typically 10-100 Angstrom.

Alternatively to monitoring the increase in fluorescence of the FRET acceptor component, it is also possible to monitor fluorescence decrease of the FRET donor component as a quantitative measurement of hybridization event.

Among all detection formats known in the art of real time PCR, the FRET hybridization probe format has been proven to be highly sensitive, exact and reliable (WO 97/46707; WO 97/46712; WO 97/46714). Yet, the design of appropriate FRET hybridization probe sequences may sometimes be limited by the special characteristics of the target nucleic acid sequence to be detected.

As an alternative to the usage of two FRET hybridization probes, it is also possible to use a fluorescent-labeled primer and only one labeled polynucleotide probe (Bernard, P. S., et al., Anal. Biochem. 255 (1998) 101-7). In this regard, it may be chosen arbitrarily, whether the primer is labeled with the FRET donor or the FRET acceptor compound.

The fluorescence can be measured during the elongation step, generating amplification curves from which, depending on the primers and/or probes used, on their Tm and on the hybridization conditions, it is possible to infer the presence of the *Proteus* species to be detected or to infer which *Proteus* species is (are) present.

FRET hybridization probes (also called HybProbes or FRET-probes) can also be used for melting curve analysis (WO 97/46707; WO 97/46712; WO97/46714). In such an assay, the target nucleic acid is amplified first in a typical PCR reaction with suitable amplification primers. The hybridization probes may already be present during the amplification reaction or be labelled subsequently. After completion of the PCR-reaction, the temperature of the sample is consecutively increased. Fluorescence is detected as long as the hybridization probe is bound to the target DNA. At the melting temperature, the hybridization probe is released from their target, and the fluorescent signal is decreasing immediately down to the background level. This decrease is monitored with an appropriate fluorescence versus temperature-time plot such that the negative of a first derivative function can be calculated. The temperature value corresponding to the obtained maximum of such a function is then taken as the determined melting temperature of said pair of FRET hybridization probes.

Point mutations or polymorphisms within the target nucleic acid result in a less than 100% complementarity between the target nucleic acid and the FRET probes, thus resulting in a decreased melting temperature. This enables for a common detection of a pool of sequence variants by means of FRET-HybProbe hybridization, whereas subsequently, different members of said pool may become discriminated by means of performing melting curve analysis.

Instead of FRET hybridization probes, Molecular Beacons may alternatively be used for melting curve analysis.

Upon the availability of Real-Time PCR and homogenous Real-Time PCR melting curve analysis, discrimination of certain types of species or strains became possible using either double stranded DNA binding dyes such as SybrGreen™I, or, alternatively, specifically designed hybridization probes hybridizing to different but similar target sequences.

In the first case, melting temperature of the generated double stranded PCR product has to be determined. Yet, this method has only limited applications since few differences cannot be monitored efficiently, because minor sequence variations only result in subtle melting temperature differences.

Alternatively, hybridization probes may be used in such a way that the melting temperature of the probe/target nucleic acid hybrid is being determined.

There are different real time PCR platforms that can be used, such as the ABI/Prism™ equipments, and in particular the LightCycler™ apparatus, all based on the same principle consisting of measuring the light emission, continually monitoring the emission peak during the melt cycle, determining and visualizing the temperatures (melting peaks) at which the labeled probes detach from the amplification products. The melting peak data are characteristic of a particular [probe:target] sequence because mismatches between probe and target affect the kinetics of melting, producing different melting peaks for each species of interest.

The LightCycler™ platform offers many advantages and in particular a gain of time and the possible use of several different sequence-specific fluorescent probe detection systems such as hybridization probes (HybProbes), TaqMan™ probes, Molecular Beacons, Scorpion probes and biprobes (SYBR Green I).

In a preferred method described herein, the HybProbe system is used, consisting of two adjacent polynucleotide probes derived from the target sequences described herein, in a head-to-tail orientation, spaced by a few nucleotides, generally 0 to 25, preferably about 1 to about 5. One of the probes is labeled at its 3' end by a donor dye, the other is labeled with an acceptor molecule at its 5' end, and is phosphate blocked at the 3' end (to prevent its acting as a primer). The donor dye is generally fluorescein, and the acceptor molecule generally LC Red 610, 640, 670 or 705.

The detection of a target sequence described hereinmay be achieved also by an internal labeled PCR strand and a detection probe located on the opposite strand. The signal is dependent on the spatial approximation of the dyes, and is dependent on the amount of the target.

When both probes are hybridized to their target sequence the emitted light of the donor is transmitted to the acceptor fluorophore by Fluorescence Resonance Energy Transfer (FRET), and the emitted fluorescence (610, 640, 670 or 705 nm) can be detected. The intensity of the emitted fluorescence increases in parallel with the target DNA, product of the amplification.

The LightCycler probes offer the advantage over the TaqMan™ probes of not requiring hydrolysis and, therefore, no additional extension of the PCR times (annealing-elongation ≤ 12 s). It is therefore possible to take advantage of the high-speed thermal cycling of the LightCycler, and complete the PCR program in only 45 minutes.

And the recent generations of real-time PCR platforms are able to monitor several probes in a single reaction, allowing the detection and/or identification of different *Proteus,* at the species level and/or the distinction of the different type of *Proteus* spacers.

Moreover, it has been shown that the methods designed for TaqMan technology can be easily converted to HybProbe technology with equivalent results (Haematologica vol. 85 (12) pp. 1248-1254, December 2000).

According to the present specification, the sets of at least two polynucleotide probes, possibly referred to as HybProbes, both HybProbes may hybridize to the same target sequence, adjacent to each other, with no more than 25 nucleotides between said 2 HybProbes, preferably with no more than 15 nucleotides, more preferably with no more than 10 nucleotides, in particular with no more than 5 nucleotides.

When there are two HybProbes, one is labeled with an acceptor fluorophore and the other with a donor such that upon hybridization of the two HybProbes with the target sequence, the donor and acceptor fluorophores are preferably within 0 to 25 nucleotides of one another, more preferably within 0 to 10 nucleotides of one another and most preferably within 0 to 5 nucleotides of one another.

When there are more than two HybProbes, at least one is labeled with an acceptor fluorophore and the others with a donor (or vice versa) such that upon hybridization of the HybProbes with the target sequence, the donor and acceptor fluorophores are preferably within 0 to 25 nucleotides of one another, more preferably within 0 to 10 nucleotides of one another and most preferably within 0 to 5 nucleotides of one another.

For detecting and/or identifying *Proteus* species, in particular *Proteus* species that are clinically relevant, a set of at least two polynucleotide probes may be used, said probes hybridizing with at least one of the target sequences selected from the group consisting of SEQ ID NOs 1 to 17, their RNA form wherein T is replaced by U, the complementary form thereof, and homologues, wherein there are preferably no more than 25 nucleotides, more preferably no more than 10 nucleotides and most preferably no more than 5 nucleotides, between said probes.

A set of probes of the invention may also consist of 3, 4, 5 ,6 ,7, 8, 9, 10, or more, probes, but it preferably consists of 2 to 5 probes.

The sets of probes listed in Table 2 and their homologues are preferred sets of the invention.

Sets of three polynucleotides, two for use as primer, the other for use as probe, may also be used. Then one of said primers and the said probe hybridize to at least one of the target sequences selected from the group consisting of SEQ ID NOs 1 to 17, their RNA form wherein T is replaced by U, the complementary form thereof, and homologues, so that there are preferably no more than 25 nucleotides, more preferably no more than 10 nucleotides an most preferably no more than 5 nucleotides between said primer and said probe.

The sets of at least two polynucleotides of the invention are used in methods for the detection and/or identification of *Proteus* species. In particular *P. mirabilis, P. vulgaris and*/*or P. penneri may be detected and*/*or identified.*

A method described herein for detection and/or identification of *Proteus* species in a sample, in particular *of P. mirabilis, P. vulgaris and*/*or P. penneri, may* comprise the steps of:
(i) optionally, releasing, isolating and/or concentrating the polynucleic acids in the sample;
(ii) optionally, amplifying the 16S-23S rRNA spacer region, or at least one target sequence, or a fragment thereof, with at least one suitable primer pair;
(iii) contacting the polynucleic acids with at least one set of at least two HybProbes that hybridize to at least one target sequence selected from the group consisting of SEQ ID NOs 1 to 17, their RNA form wherein T is replaced by U, the complementary form thereof, any homologues, and a fragment of at least 10 and preferably at least 20 contiguous nucleotides thereof;
(iv) detecting the hybrids formed in step (iii);
(v) inferring the presence of *Proteus* species, or identifying the *Proteus* species in the sample from the differential hybridization signals obtained in step (iv).

For example, a primer pair used in the amplification step is any combination of a forward primer derived from any of the polynucleotides represented by SEQ ID NO 53 or 61 or their homologues, and a reverse primer derived from any of the polynucleotides represented by SEQ ID NO 44, 58 or 59 or their homologues.

For example, a set of two HybProbes used in the hybridization step can be any combination of the HybProbe represented by SEQ ID NO 22 with any of the HybProbes represented by SEQ ID NOs 37, 38 and 39, or their homologues.

The HybProbe represented by SEQ ID NO 22 can be fluorescein labeled and the others can be either LCR610, LCR640, LCR670 or LCR705 labeled

One of the advantages of the HybProbes system resides in the fact that it allows the detection of sequence variation, including mutations, polymorphisms and other variant nucleic acid species, based on the following molecular concept: one of the HybProbe is a tightly binding "anchor probe" whereas the adjacent "sensor probe" spans the region of sequence variation. During melting of the final PCR product, the sequence alteration is detected as a change in the melting temperature (Tm) of the sensor probe.

For example, if the sample contains only SEQ ID NO 1, using HybProbes that specifically hybridize to said SEQ ID NO 1 would generate a single melting peak. If there is also a homologue in the sample, using the same two HybProbes would generate two peaks, as far as there is at least one mismatched base which generally induces a temperature shift easily observable.

Depending on the format of the probes used for the detection of the products of the amplification, on the polynucleotides selected (or designed), on their Tm and on the hybridization conditions, the fluorescence may be measured during the amplification step, generating then amplification curves, or after the amplification step, for a melting curve analysis, generating melting curves.

Thus the signal(s) obtained may be visualized in the form of amplification curves or in the form of melting curves, from which it is possible to infer the presence of *Proteus* species, and/or to infer which one(s) of the *Proteus* species is/are present.

In particular, a method for detection and/or identification of *Proteus species* in a sample comprises also the steps of
(i) if need be releasing, isolating and/or concentrating the polynucleic acids in the sample, and
(ii) amplifying at least one of the target sequences selected from the group consisting of SEQ ID NO 1 to 17, their RNA form wherein T is replaced by U, the complementary form thereof, any homologues, and a fragment of at least 20 contiguous nucleotides thereof, with a pair of primers one of which is labeled,
(iii) contacting the polynucleic acids with at least one HybProbe that hybridize, adjacent to said labeled primer with less than 25 nucleotides in between, to said target sequence(s),
(iv) detecting the hybrids formed, and
(v) inferring the presence of *Proteus* species, and/or identifying the *Proteus* species in the sample from the signals obtained in step (iv).

According to the present specification, the method described herein using the HybProbes system, may be adapted for the detection and identification of one or several *Proteus species,* allowing its/their distinction from other *Proteus* species.

In particular, a method described herein using the HybProbes system, may be adapted for the detection and identification of *Proteus mirabilis,* allowing its distinction from other *Proteus* species.

Then, in the amplification step, suitable primers are primer pairs that specifically amplify the target sequence(s) selected from a group consisting of SEQ ID NOs 1 to 10, their RNA form wherein T is replaced by U, the complementary form thereof and homologues.

In the hybridization step, the HybProbes should hybridize specifically for example to any of SEQ ID NO 21 to 24, 27 to 29, 37 to 39,47 to 49, 51, 54, 55, and 65 to 67 or to their RNA form wherein T is replaced by U, or to the complementary form thereof.

Therefore, *Proteus mirabilis* strains can be unequivocally distinguished from all other organisms examined by melting curve analysis.

No relevant signals are obtained with *non-Proteus* species or human genomic DNA.

A preferred set of 2 HybProbes consists of SEQ ID NO 24 or homologues and SEQ ID NO 39 or homologues.

This set of HybProbes consisting of SEQ ID NO 24 and 39 is able to *Proteus mirabilis* with a high sensitivity.

In addition, the method described herein using the HybProbes system, may also be adapted for the detection and/or identification of *Proteus vulgaris* or *Proteus penneri,* allowing the distinction of the first or the latter from other *Proteus* species.

Then, for the detection and/or identification of *Proteus vulgaris,* in the amplification step, suitable primers are primer pairs that specifically amplify the target sequence(s) selected from a group consisting of SEQ ID NOs 11 to 13, their RNA form wherein T is replaced by U, the complementary form thereof and homologues.

In the hybridization step, the HybProbes should hybridize specifically for example to any of SEQ ID NO 18 to 20, 56 and 57 or to their RNA form wherein T is replaced by U, or to the complementary form thereof.

According to the present specification, each polynucleotide listed in Table 4, corresponding to SEQ ID NO 18 to SEQ ID NO 67 and any of their homologues, may be used in any methods described herein as a primer and/or as a probe, alone or in combination.

A second embodiment based also on a hybridization method is the Line Probe Assay technique. The Line Probe Assay (LiPA) is a reverse hybridization format (Saiki et al. (1989). Proc Natl Acad Sci. USA 86:6230-6234) using membrane strips onto which several polynucleotide probes (including negative or positive control polynucleotides) can be conveniently applied as parallel lines. The LiPA technique, as described by Stuyver et al. ((1993) J. Gen Virology 74:1093-1102) and in European patent EP 637342, provides a rapid and user-friendly hybridization test. Results can be read within 4 h. after the start of the amplification. After amplification during which usually a non-isotopic label is incorporated in the amplified product, and alkaline denaturation, the amplified product is contacted with the probes on the membrane and the hybridization is carried out for about 1 to 1,5 h. Consequently, the hybrids formed are detected by an enzymatic procedure resulting in a visual purple-brown precipitate. The LiPA format is completely compatible with commercially available scanning devices, thus rendering automatic interpretation of the results possible. All those advantages make the LiPA format liable for use in a routine setting.

The LiPA format is an advantageous tool for detection and/or identification of pathogens at the species level but also at higher or lower taxonomical levels. For instance, probe-configurations on LiPA strips can be selected in such a manner that they can detect the complete genus of *Proteus* or can identify species within the genus (e.g. *P. mirabilis, P. vulgaris* and/or *Proteus penneri,* etc) or can in some cases even detect subtypes within a species.

The ability to simultaneously generate hybridization results with a large number of probes is another benefit of the LiPA technology. In many cases the amount of information which can be obtained by a particular combination of probes greatly outnumbers the data obtained by using single probe assays. Therefore the selection of probes on the membrane strip is of utmost importance since an optimized set of probes will generate the maximum of information possible.

These probes can be applied to membrane strips at different locations and the result is interpreted as positive if at least one of these probes is positive. Alternatively these probes can be applied as a mixture at the same location, hereby reducing the number of lines on a strip. This reduction may be convenient in order to make the strip more concise or to be able to extend the total number of probes on one strip.

Another approach is the use of degenerate probes, which can considerably simplify the manufacturing procedures of the LiPA-strips.

Still another approach are chimeric-probes comprising two oligonucleotides described herein. For example, sequences of SEQ ID NO 37 and 55 are both required to detect the two types of ITS form *P. mirabilis* In this alternative a probe can be synthesized having the nucleotide sequence of the first SEQ ID NO followed by the nucleotide sequence of the second. This probe will have the combined characteristics of the two probes sequences of SEQ ID NO 37 and 55.

These two approaches can also be used in any embodiments or methods described herein.

By virtue of the above-mentioned properties the LiP A system can be considered as an efficient format for a hybridization method wherein several organisms need to be detected simultaneously in a sample.

However, it should be clear that any other hybridization assay, whereby different probes are used under the same hybridization and wash conditions can be used for the above-mentioned detection and/or selection methods. For example, it may be possible to immobilize the target nucleic acid to a solid support, and use mixtures of different probes, all differently labeled, resulting in a different detection signal for each of the probes hybridized to the target. And nowadays many different supports are available.

As an example, the procedure to be followed for the detection of one or more *Proteus* species in a sample using the LiPA format is outlined below:
- First, and if necessary, the nucleic acids present in the sample are made available for amplification and/or hybridization.
- Optionally, the nucleic acids are amplified with one or another target amplification system. Usually, amplification is needed to enhance the subsequent hybridization signal.
- Thirdly, eventually after a denaturation step, the nucleic acids present in the sample or the resulting amplified product are contacted with LiP A strips onto which one or more probes, allowing the detection of the organisms of interest, are immobilized, and hybridization is allowed to proceed.
- Finally, eventually after having performed a wash step, the hybrids are detected using a convenient and compatible detection system. From the hybridization signal(s) or pattern(s) observed the presence or absence of one or several organisms screened for in that particular biological sample can be deduced.

Universal primers located in the conserved flanking regions of the rRNA spacer, i.e. in the 16S gene and the 23S gene, can be used.

For some applications it may be appropriate to amplify not different bacteria present in the sample but more specifically *Proteus* species.

According to the present specification, a method for detection and/or identification of *Proteus species* in
a sample, may comprise the steps of:
(i) if need be releasing, isolating and/or concentrating the polynucleic acids present in the sample;
(ii) if need be amplifying the 16S-23S rRNA spacer region, or a part of it, with at least one suitable primer pair;
(iii) contacting the polynucleic acids with at least one probe that hybridizes to the target sequence consisting of SEQ ID NO 1 or 17, or of the RNA form of said SEQ ID NO 1 or 17 wherein T is replaced by U, or of the complementary form thereof, or of any homologues, or of a fragment of at least 10 and preferably at least 20 contiguous nucleotides thereof;
(iv) detecting the hybrids formed in step (iii);
(v) detecting and/or identifying the micro-organism(s) present in the sample from the differential hybridization signals obtained in step (iv).

The part of the ITS mentioned in the step of amplification, is a polynucleotide comprising the target sequence, or the target sequence itself, the target sequence consisting of any of SEQ ID NO 1 to 17, or of their RNA form wherein T is replaced by U, or of the complementary form thereof, or of any homologues, or of a fragment of at least 20 contiguous nucleotides thereof.

Preferentially, the present specification discloses a method as described above wherein at least 2 micro-organisms are detected simultaneously.

A set of probes as described in step (iii) comprises at least two, three, four, five, six, seven, eight, nine or more probes described herein.

In a preferred method described herein, set of probes as described in step (iii) comprises at least two probes.

Preferred probes are polynucleotides of SEQ ID NO 18 to 67, their RNA form wherein T is replaced by U, the complementary form thereof, any homologues, and fragments of about 10 contiguous nucleotides thereof, with the proviso that the nucleic acid molecule ATACGTGTTATGTGC is excluded, more preferred are fragments of about 20 contiguous nucleotides thereof.

The present specification also describes a method as described above, wherein the probes as specified in step (iii) are combined with at least one other probe, preferentially also from the 16S-23S rRNA spacer region, enabling the simultaneous detection of different pathogenic bacteria liable to be present in the same sample.

Preferred probes are designed for attaining optimal performance under the same hybridization conditions so that they can be used in sets for simultaneous hybridization; this highly increases the usability of these probes and results in a significant gain in time and labor.

A kit containing any of the polynucleotides described hereinis also disclosed.

According to the present specification, a kit may comprise the following components:
- at least one polynucleotide hybridizing to the target sequence consisting of any of SEQ ID NO 1 to 17, their RNA form wherein T is replaced by U, the complementary form thereof, or homologues thereof;
- a hybridization buffer, or components necessary for producing said buffer.

According to the present specification, a preferred kit may comprise
- at least one set of two HybProbes hybridizing, adjacent to each other with less than 25 nucleotides, preferably less than 5 nucleotides, to the target sequence consisting of any of SEQ ID NO 1 to 17, their RNA form wherein T is replaced by U, the complementary form thereof, or any homologues thereof;
- a hybridization buffer, or components necessary for producing said buffer.

To conclude, using the *Proteus* ITS as target, it is possible to design probes to be used in different detection and/or identification methods.

With the real time PCR method, on the one hand it is possible to detect and identify the *Proteus* genus - in particular *P. mirabilis, P. vulgaris, and P. penneri -* using one single HybProbe set generating one single melting peak in the LightCycler system (example 4).

On the other hand, a species-specific signal can be obtained by the presence of one specific melting peak for one particular species *(P. mirabilis* in example 3), or by the presence of a peak at a Tm that is specific for a particular species (*see P. vulgaris* and *P. penneri* in examples 5 and 6).

Also sequencing the complete ITS region and comparing it to a reference sequence as given here, can be used as a method to detect and identify *Proteus* species (example 7).

The preceding description or the Examples which follow should not be construed as limiting the invention to the embodiments specifically disclosed therein.

### EXAMPLES

For the examples described below, the 16S-23S internal transcribed spacer (ITS) was amplified using primers designed in conserved regions of the 16S rRNA and 23S rRNA, respectively.

### Example 1: LightCycler protocol

DNA was prepared according to standard methods, and about 10⁴ genome equivalents were used as target for amplification.

A sample was flagged positive if a quantification curve and a melting peak were present for that sample.

The probes were designed to work as HybProbes in the LightCycler v1.2 (software v4) enabling a real-time fluorescence PCR detection.

One HybProbe was labeled at its 3' end with a fluorescein dye, while the neighbouring HybProbe was labeled at its 5' end with a LC-red 640 or LC-red 705 dye.

Following the instructions of the manufacturer of the kit LC-FastStart DNA Master Hybridization Probes (cat. No 3 003 248 or No 2 239 272):
- any sample material suitable for PCR. in terms of purity, concentration, and absence of inhibitors can be used;
- the primers should be at a final concentration of 0,3 to 1 µM each;
- the HybProbes at a final concentration of 0,2 µM each, or double;
- the concentration of MgCl₂ should be optimized, and may vary from 1 to 5 mM;
- and a negative control should be run.

The amplification and melting conditions are described herein after. The LC software version 4 was used. The quantification settings were F2/back F1 (samples). For the baseline adjustment the arithmetic mode was used. The crossing point (Ct) calculation was based on the second derivative maximum. The calculation method for the melting peak was polynomial. The peak area was used to calculate the Tm.

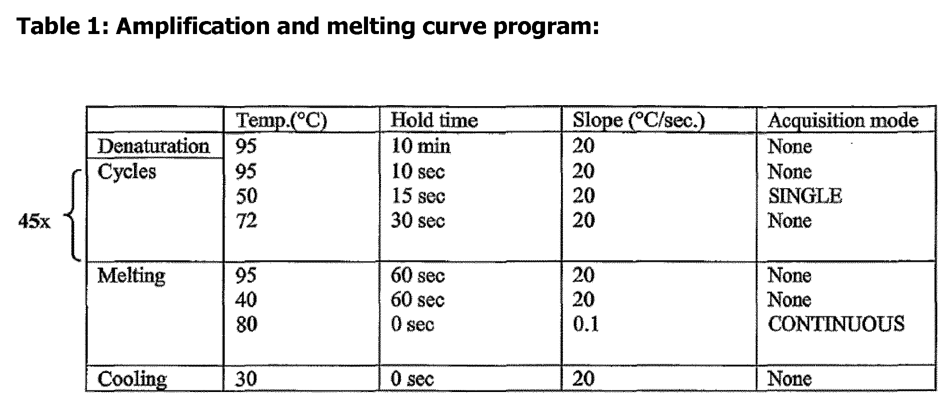

### Example 2: Different sets ofHybProbes

In this example, one HybProbe was labeled at its 3' end with a fluorescein dye, while the neighboring HybProbe was labeled at its 5' end with LC-Red 640 or LC-Red 705 dye.

The same Lightcycler protocol as described in example 1 was applied.

**Table 2: Results of different combinations tested**

| SEQ ID NOs Fluoresecin labeled | SEQ ID NOs LC-Red labeled | Design goal | Strains detected / strains tested | | | | Preferred/ most preferred |
|---|---|---|---|---|---|---|---|
| | | | *P. mirabilis* | *P. vulgaris* | *P. penneri* | Other bacteria | |
| 21 | 37 | *P. mirabilis* specific | 17/17 | 0/1 | 0/2 | - | ++ |
| 23 | 37 | *P. mirabilis* specific | 17/17 | 0/1 | 0/2 | - | ++ |
| 21 | 38 | *P. mirabilis* specific | 2/2 | 0/1 | 0/1 | - | + |
| 23 | 38 | *P. mirabilis* specific | 2/2 | 0/1 | 0/1 | - | + |
| 24 | 37 | *P. mirabilis* specific | 4/4 | - | - | - | + |
| 24 | 38 | *P. mirabilis* specific | 4/4 | - | - | - | + |
| 24 | 39 | *P. mirabilis* specific | 42/42 | 0/3 | 0/3 | 0/56 | ++ |
| 22 | 37 | *P. mirabilis* specific | 42/42 | 0/3 | 0/3 | 0/56 | ++ |
| 22 | 38 | *P. mirabilis* specific | 4/4 | - | - | - | + |
| 22 | 39 | *P. mirabilis* specific | 4/4 | - | - | - | + |
| 25 | 40 | *Proteus* genus | 2/2 | 1/1 | 1/1 | - | + |
| 25 | 41 | *Proteus* genus | 2/2 | 1/1 | 1/1 | - | + |
| 26 | 40 | *Proteus* genus | 2/2 | 1/1 | 1/1 | - | + |
| 26 | 41 | *Proteus* genus | 2/2 | 1/1 | 1/1 | - | + |
| 27 | 42 | *Proteus* genus | 2/2 | 1/1 | 1/1 | - | + |
| 28 | 42 | *Proteus* genus | 2/2 | 1/1 | 1/1 | - | + |
| 29 | 42 | *Proteus* genus | 2/2 | 1/1 | 1/1 | - | + |
| 30 | 43 | *Proteus* genus | 19/19 | 2/2 | 2/2 | 0/8 | ++ |
| 30 | 44 | *Proteus* genus | 2/2 | 1/1 | 1/1 | - | + |
| 31 | 43 | *Proteus genus* | 42/42 | 3/3 | 3/3 | 0/56 | ++ |
| 31 | 44 | *Proteus* genus | 2/2 | 1/1 | 1/1 | - | + |
| 32 | 45 | *Proteus* genus | 2/2 | 1/1 | 1/1 | - | + |
| 32 | 46 | *Proteus* genus | 2/2 | 1/1 | 1/1 | - | + |
| 33 | 45 | *Proteus* genus | 19/19 | 2/2 | 2/2 | 0/8 | ++ |
| 33 | 46 | *Proteus* genus | 2/2 | 1/1 | 1/1 | | + |

### Example 3: P. mirabilisspecific HybProbes

The HybProbes represented by SEQ ID NO 24 and SEQ ID NO 39 were used in a LightCycler protocol as described in example 1. The first (SEQ ID NO 24) was fluorescein labeled and the second (SEQ ID NO 39) was LC-Red 640 labeled.

The same Lightcycler protocol as described in example 1 was applied, and the sample used contained one of the *P. mirabilis* strains. One specific melting peak at 53°C was observed.

The sensitivity of this HybProbe set was evaluated using 42 *P. mirabilis* strains (10 originating from West-Europe, 10 from the UK, 10 from South-Europe, 10 from the United States, and 2 from Japan). All *P. mirabilis* strains had a visible quantification curve with Ct values varying from 19.95 to 22.81.

A melting peak: of 53°C (STDEVA 0.60°C) was observed for all *P. mirabilis* strains tested, showing a 100% sensitivity for *P. mirabilis* with this HybProbes set.

In order to test specificity, 3 *P. vulgaris* strains and 3 *P. penneri* strains were tested. No quantification curve and no melting curves were obtained, showing a specificity of 100% having regard to the other *Proteusspecies* clinically relevant.

Besides these *Proteus* species, a large panel of other organisms was tested (see
Table 3) and a further experiment was done with human DNA. Neither the human DNA nor the microorganisms tested gave any quantification curve or any melting peak, confirming the HybProbes specificity of 100%.

**Table 3: list of microorganisms tested for specificity.**

| | |
|---|---|
| *Acinetobacter baumannii* | *Bartonella henselae* |
| *Aspergillus fumigatus* | *Bordetella pertussis* |
| *Candida albicans* | *Borrelia burgdorferi* |
| *Candida glabrata* | *Burkholderia cepacia* |
| *Candida krusei* | *Campylobacter jejuni* |
| *Candidia parapsilosis* | *Cardiobacterium hominis* |
| *Candida tropicalis* | *Citrobacter freundii* |
| *Enterobacter aerogenes* | *Clastridium perfringens* |
| *Enterobacter cloacae* | *Corynebacterium jeikeium* |
| *Enterococcus faecalis* | *Cryptococcus neoformans* |
| *Enterococcus faecium* | *Gemella haemolysans* |
| *Escherichia coli* | *Histoplasma capsulatum* |
| *Klebsiella oxytoca* | *Haemophilus influenzae* |
| *Klebsiella pneumoniae* | *Legionella pneumophila* |
| *Pseudomonas aeruginosa* | *Listeria monocytogenes* |
| *Serratia marcescens* | *Moraxella (Branhamella) catarrhalis* |
| *Staphylococcus aureus* | *Morganella morganii* |
| *Staphylococcus epidermidis* | *Mycobacterium fortuitum* |
| *Staphylococcus haemolyticus* | *Mycobacterium tuberculosis* |
| *Stenotrophomonas maltophilia* | *Mycoplasma pneumoniae* |
| *Streptococcus* s*anguinis "Sanguinis"* groups | *Neisseria meningitidis* |
| *Streptococcus agalactiae* | *Pantoea agglomerans* |
| *Streptococcus pneumoniae* | *Peptostreptococcus magnus* |
| *Streptococcus pyogenes* | *Porphyromonas gingivalis* |
| *Actinobacillus actinomycetemcomitans* | *Prevotella denticola* |
| *Aeromonas hydrophila* | *Propionibacterium acnes* |
| *Bacillus cereus* | *Salmonella enterica venteritidis* |
| *Bacteriodes fragilis* | *Yersinia enterocolitica* |

This HybProbes set is able to detect and *identify P. mirabilis* in a specific manner.

### Example 4: HybProbes for Proteusspecies

Four samples containing respectively two strains *of P. mirabilis* (each strain in one sample), one *of P. penneri,* and one *of P. vulgaris* were tested.

The HybProbes represented by SEQ ID NO 30 and SEQ ID NO 44 were used in a LightCycler protocol as described in example 1.

Each strain generated a quantification curve and one melting peak at 55°C was observed.

Therefore, this HybProbes set is able to detect and identify different *Proteus* species, in particular the *Proteus* species that are clinically relevant.

### Example 5: HybProbes for distinguishing P. pennerifrom other Proteusspecies.

Four samples containing respectively two strains *of P. mirabilis* (each strain in one sample), one *of P. penneri,* and one *of P. vulgaris,* were tested with another set of HybProbes.

The HybProbes represented by SEQ ID NO 27 and SEQ ID NO 42 were used in a LightCycler protocol as described in example 1.

Each strain generated a quantification curve. After a melting curve analysis, *P*. *penneri* showed a melting peak at 52.5°C. The three others showed a melting peak at 56°C.

This HybProbes set allows therefore to distinguish and identify *P. penneri* from the other *Proteus* species.

### Example 6: HybProbes for distinguishing P. vulgaris from other Proteus species.

Four samples containing respectively two strains *of P. mirabilis* (each strain in one sample), one *of P. penneri,* and one *of P. vulgaris* were tested with another set of HybProbes.

The HybProbes represented by SEQ ID NO 32 and SEQ ID NO 45 were used in a LightCycler protocol as described in example 1.

Each strain generated a quantification curve. After a melting curve analysis, *P*. *vulgaris* showed a melting peak of 54.5°C. The two others showed a melting peak at 52.5°C.

This HybProbes set allows therefore to distinguish and identify *P. vulgaris* from the other *Proteus* species.

Having regard to the ITS sequences of each species, only one melting peak at 54.5°C was expected.

The result obtained means that the strain *of P. vulgaris* tested contains a polymorphism in its ITS sequence which is responsible for the shift observed in the Tm.

### Example 7: Detection and identification of Proteus spp. by ITS nucleotide sequence determination.

A sample was received without a clear indication of the *Proteus* species it was supposed to contain.

The ITS region of the species to be determined was amplified using universal primers located in the 16S and 23S.

The amplicons were cloned into the pGEM-T vector (promega) and the ITS nucleotide sequences were derived according to the dideoxy-chain terminating chemistry using primers located in the plasmid vector.

Both a spacer containingtRNA^{glu} and tRNA^{ile-ala} were found.

These ITS sequences were submitted to sequence analysis, and compared with the other spacers already sequenced.

The nucleotide sequence of the tRNA^{glu} spacer from the sample to be identified was completely identical to the tRNA^{glu} consensus spacer nucleotide sequence of *P*. *mirabilis* represented by SEQ ID NO 4.

The nucleotide sequence of the tRNA^{ile-ala} spacer from this sample differed in 3 base pairs out of 702 (99.4% homologies) when compared to the consensus nucleotide sequence of the tRNA^{ile-ala} spacer *of P. mirabilis* represented by SEQ ID NO 6.

In view of the high degree of homology, it could be inferred that the sample contained *P. mirabilis*

### Example 8: HybProbes for distinguishing the three Proteus species clinically relevant.

A set of three HybProbes represented by SEQ ID NO 50, SEQ ID NO 51 and SEQ ID NO 52 were designed for a LightCycler protocol as described in example 1, for samples containing respectively *P. mirabilis, P. penneri, and P. vulgaris*

The first HybProbe (SEQ ID NO 50) Fluorescein labeled, and the two others (SEQ ID NO 51 and SEQ ID NO 52) LC-Red labeled, allow the distinction *of P. mirabilis* from *P. vulgaris* and *P. penneri* by the means of melting curves, the one representing *P. mirabilis* having a melting peak at 63°C and the two others at 67°C.

**Table 4**

| **SEQ ID NO** | **Sequences** |
|---|---|
| 1 | Cctaagagatacgtgtatgtgmagtgctcacacagattgtctgatgaagaacgagcaaaagcgcgtctgcgaagctgacaraagtcccccttcgtctagaggcctaggacaccgccctttcacggcggtaacaggggttcgaatcccctaggg gacgccaatgcgcggtatgagtgaaaggcgtaccacttatctgacgagagtcagagaataactaagctaattcaaaygagttatcttayttattatgctcttaacaatctggaacaagctgaaaaattgaaaacaaatcaatatatcamcgagrtat attggtgagtctctcaaaatctcaaatctgaaaagtactcttcagaaaggatatgcgagcaaaatgatttcaaggcggacagcgcacagcaagcgcagcatacttaaagtatgtgagcattgcgagcactgcccaacacagaaatcatgaagcg cagcaatccgtttaaaagacactttcgggtgtga |
| 2 | Cctaagagatacgtgttatgtgaagtgctcacacagaatgctgatgaagaacgagcaaaagcgcgtctgcgaagctgacagaagtcccctcgtctagaggctaggacaccgcccttcacggcggtaacagggttcgaatccctaggg gacgccaatgcgcggtatgagtgaaaggcgtaccacttatctgacgaaagtcagagaataactaagctaattcaaacgagttatcttacttattatgctctttaacaatctggaacaagctgaaaaattgaaaacaaatcaatatatcaacgaggtatt tgatgagtctctcaaaatctcaaatctgaaagtactcttcagaaaggatatgcgagcaaaatgatttcaaggcggacagcgcacagcaagcggcagcatacttaaagtatgtgagcattgcgagcactgcccaacgaagaaatgtaatctgcgca gccatcaccacccagatagtcttgaaagagacactttcgggttgtga [?] |
| 3 | Cctaagagatacgtgttatgtgmagtgctcacacagattgtctgatgaagaacgagcaaaagcgcgtcgcgaagctgacaraagtcccttcgtctagaggcctaggacaccgcccttcacggcggtaacagggttcgaatcccctaggg gacgccaatgcgcggtatgagtgaaaggcgtaccacttatctgacraragtcagagcataactaagctaattcaaaygagttatcttayttattatgctctttaacaatctggaacaagctgaaaaattgaaaacaaatcaatatatcamcgaggtat attgrtgagtctctcaaaatctcaaacttgaatgtgtttgacatcaaagtgggatgagcgagcaattacagttcgaggcggacagcgcrcagcaagcgcagcatacttwwgtatgtgagcattgcgagcactgcccaacgaagaaatgtaatc tgcgcagccatcaccacctagatagtctttgaaagagacactttcgggttgtga [?] |
| 4 | Cctaagagatacgtgtatgtgmagagtgctcacacagattgtctgatgaagaacgagcaaaagcgcgtctgcgaagctgacaraagtcccttcgtctagaggcctaggacaccgcccttcacggcggtaacagggttcgaatcccctaggg gacgccaatgcgcggtatgagtgaaaggcgtaccacttatctgacraragtcagagaataaytaagctaattcaaaygagttatcttayttattatgctctttaacaatctggacaagctgaaaaattgaaacaaatcaatatatcamcgaggtat attgrtgagtctctcaaaatctcaaaccttaaagktttgtcacrcaaagtttatctttgaaaragacacttcgggtgtga C[?] |
| 5 | Cctaagagatacgtgttatgtgaagtgctcacacagattgtctgatgaagaacgagcagagataccggtataggcttgtagctcaggtggttagagcgcacccctgataagggtgaggtcggtggttcaagtccactcaggcctaccactttcct ttatgctgtgtgtgaagcaactcgtttacattaagtaaacttcgttactccacgccttgcctaaagaaaaagcttcttattataagaagaaaaaggagtggttatacrggtattraaacattatggggctatagctcagctgggagagcgcctgccttgc acgcaggaggtcagcggttcgatcccgcttagctccaccataatctcttgratataaaacaatgattcagagtatattaggaatagtatactgygaattattatgctctttaacaatctggaacaagctgaaaaattgaaaacaaatcaatatatcaccg aggtatattgatgagtctctcaaaatctcaaactttgaatgtgttttgacatcaaagtgggatgagcgagcaatttacagttcgaggcggacagcgcacagcaagcgcagcatacttaagtatgtgagcattgcgagcactgcccaacgaagaaat gtaatctgcgcagccatcrccacccagatagtcttgaaagagacacttcgggtgtga [?] |
| 6 | Gctaagagaacgcgtaggtgtaggtgctcacacagatgtcgatgaagatcgagcagagatacggatatggctgtagctcaggggttagagcgcacccctgataagggtgaggtcggtggttcaagtccactcaggcctaccaaatcgt attgatactgcgttgtgaataaactcgttactgattgtaaacttcgttgattcacgccttgtctcactacgattcactcattatagtaaaggyactccctttaaragagtaartaatcggtattaaaacattatggggctatagctcagctgggagagcgc ctgccttgcacgcaggaggtcagcggttcgatcccgcttagctccaccataatctcttgaatataaacaatgatcagagtatattaggaatagtatactgtgaattattatgctctttaacaatctggaacaagctgaaaaattgaaaacaaatcaata tatcaccgaggtatattgatgagtctctcaaaatctcaaacttgaatgtgtttgacatcaaagtgggatgagcgagcaatttacagttcraggcggacagcgcacagcaagcgcagcatacttaagtatgtgagcattgcgagcactgcccaacg aagaaatgtaatctgcgcagccatcaccacccagatagtcttgaaagagacacttcgggttgtga |
| 7 | Cctaagagatacgtgttatgtgcagtgctcacacagattgtctgatgaataacgagcagagataccggtatagcttgtagctcaggtggttagagcgcacccctgataagggtgaggtcggtggttcaagtccactcaggcctaccactttttcctt tatgcgtgtgttgtgaagcaactcgtttacattaagtaaacttcgttactccacgccttgcctaaggaaaaagcttcttaatataaaagaaaaaggagtggttatacgggtattaaaacattatgggctatagctcagctgggagagcgcctgccttgc acgcaggaggtcagcggttcgatcccgcttagctccaccataatctcttgaatataaaacaatgattcagagtatattaggaatagtatactgcgaattattatgctctttaacaatctggaacaagctgaaaattgaaaacaaatcaatatatcaccg aggtatattggtgagtctctcaaaatctcaaatctgaaaagtactcttcagaaaggatatgcgagcaaaatgatttcaaggcggacagcgcacaagcaagcgcagcatacttaaaagtatgtgagcattgcgagcactgcccaacaccagaaatc atgaacgcgttcaatccc[?]ttttaaaaagacactttccggtctgtga |
| 8 | cctaagagatacgtgttatgtgmagtgctcacacagattgctgatgaagaacgagcagagataccggtataggcttgtagctcaggtggttagagcgcacccctgataagggtgaggtcggtggttcaagtccactcaggcctaccactttcct ttatgctgtgttgtgaagcaactcgttacattaagtaaacttcgttactcccacgccttgcctaaagaaaaagcttcttattataagaagaaaaggagtggttatacrggtattraaacattatggggctatagctcagctgggagagcgcctgccttgc acgcaggaggtcagcggttcgatcccgcttagctccaccataatctcttgratataaaacaatgattcagagtatattaggaatgtatactgygaattattatgctcttaacaatctggaacaagctgaaaaattgaaacaaatcaatatatcamc gaggtatattggtgagtctctcaaaatctcaaaccttaaagkttgtcacgcaagttatcttgaaaragacacttcgggttgtga |
| 9 | cctaagagatacggttatgtgaagtgctcacacagattgtctgatgaagaacgagcagagatacggatatggcttgtagctcaggtggttagagcgcaccctgataagggtgaggtcggtggttcaagtccactcaggcctaccaaatcgta ttgatactgcgttgtgaataaactcgttactgattgtaaactcgttgattcacgccttgtctcactacgattcactcattataattaaaggcattcccttaa[?]agagtaa[?]taatcggtattaaaacattatggggctatagctcagctgggagagcgcct gccttgcacgcaggaggtcagcggttcgatcccgcttagctccaccataatctcttgaatataaaacaatgattcagagtatattaggaatagtatactgcgaattattatgctctttaacaatctggaacaagctgaaaattgaaacaaatcaatat atcaacgaggtatattggtgagtctctcaaaatctcaaaccttaaagtttgtcacgcaagtttatctttgaaagagacactttcgggttgtga |
| 10 | cctaagagatacgtgttatgtgcacgtgctcacacagattgtctgatgaagaacgagcagagataccggtataggcttgtagctcaggtggttagagcgcacccctgataagggtgaggtcggtggttcaagtccactcaggcctaccactttcc tttatgcgtgtgttggaagcaactcgttacattaagtaaacttcgttactccacgccttgcctaagaaaaaagcttcttattataattataagaagaaaaggagtggttatacgggtattaaaacattatgggcctatagctcagctgggagagcgcc tgcctgcacgcaggaggtcagcggttcgatcccgcttagctccaccataatctctgaatataaaacaatgattcagagtatattaggaatagtatactgtgaattattatgctctttaacaatctggaacaagctgaaaattgaaacaaatcaata tatcaacgaggtatattggtgagtctctcaaaatctcaaatctgaaaagtactcttcagaaaggtatgcgagcaaaatgatttcaaggcggacagcgcacagcaagcgcagcatacttagtatgtgagcattgcgagcactgcccaacacagaa atcatgaagcgcagcaatccgttttaaaagacacttcgggtgtga |
| 11 | cctaagagatacgtgttatgtgcagtgctcacacagattgtctgatgaagaatgagcagaaataccggtataggcttgtagctcaggtggttagagcgcaccccctgataagggtgaggtcggtggttcaagtccactcaggcctaccaaatcgtat tgatactgcgttgtgaaatcactcgttactgatgtaaacttcgtgacttcacgcctgtctcactgcgattggtcaattcttacttaaaggaagacttccaataagaaagaaacctgagaaataaaaacggtattaagaatgcattatggggctatag ctcagctgggagagcgcctgccttgcacgcaggaggtcagcggttcgatcccgcttagctccaccataatctcttgaatataaaataataattcagagtatattagcaatagtatactgcgaattaytttgctctttaacaatctggaacagctgaaa aattgaaaacaaatcaatatatcaccgaggtatattgatgagtctctcaaaatctcaaacttgaatgtgttttcgacatcgaagtgggatgagcgagcaatttacagttcgaggcggccagccgcacagtcagcgcaacatacattagtatgtgagc atggcgaacnnngcccaacgacgaaatgtaatctgcacagccatcaccacccagacgtcattaagaagaaacatcttcgggtgtga cctaagagatacgtgttatgtgcagtgctcacacagattgtctgatgaagaatgagcagaaataccggtataggcttgtagctcaggtggttagagcgcaccccctgataagggtgaggtcggtggttcaagtccactcaggcctaccaaatcgtat |
| 12 | cctaagagatacgtgttatgtgcagtgctcacacagattgtctgatgaagaatgagcagaaatacggatataggcttgtagctcaggtggttagagcgcacccctgataagggtgaggtcggtggttcaagtccactcaggcctaccaaatcgtat tgatactgcgttgtgaaatcactcgttactgatgtaaacttcgtgacttcacgccttgtctcactgcgattggctcaattcttacttaaaggaagacttccaataagaaagaacctgagaaataaaaacggtattaaagaatgcattatggggctatag ctcagctgggagagcgcctgccttgcacgcaggaggtcagcggttcgatcccgcttagctccaccataatctcttgaatataaataaataattcagagtatattagcaatagtatactgcgaattatttgctctttaacaatctggaacaagctgaaa attgaaaacaaatcaatatatcaccgaggtatattgatgagtctctcaaaatctcaaacttgaagtgtgaaactccaagacattcgtcttcgagaggaacaccttcgggttgtga |
| 13 | cctaagagatacgtgttatgtgcagtgctcacacagattgtctgatgaaggacgagcaaaagcgcgtctgcgaagctgactgaagtcccttcgtctagaggcctaggacacgcccttcacggcggtaacagggttcgaatcccctagggg acgccaattgcgcggtatgagtgaaaggcgtaccacactatagtctgatgcaaatcagagaatagttagatatttagcaagttatttaactattatgctctttaacaatctggaacaagctgaaaattgaaaacaaatcaatatatcaccgaggt atattgatgagtctctcaaaatctcaaacttgaatgtgtttttcgacatcgaagtgggatgagcgagcaatttacagttcgagggcggccagcgcacagtcagcgcaacatacattagtatgtgagcatggcgagcactgcccaacgacgaaatgta atctgcacagccatcaccacccagacgtcatyaagagaaacatcttcggttgtga |
| 14 | cctaagagatacgtgttatgtgyagtgctcacacagattgtctgatgaagaacgagcaaaagcgcgtctgcgaagctgactgaagtcccttcgtctagaggcctaggacaccgccctttcacggcggtaacaggggttcgaatcccctagggg acgccaattgcgcggtatgagtgaaaggcgtaccacactatgtctgatgcaaatcagggaatagttaagataattcgatgagtattttacctattatgctctttaacaatctggaacaagctgaaaaattgaaacaaatcaatatatcaccgaggt atattgatgagtctctcaaaatctcaaacttgaatgtgtttttcgacatcaaagtgggatgagcgagcaatttacagttcgaggcggccagcgcacagccagcgcaacatacataagtatgtgagcatggcgagcactgcccaacaacgaaatgt aatctgcgcagccatcaccacccagatgtcttcaagaaaagacaccttcgggttgtga |
| 15 | cctaagagatacgtgttatgtgcagtgctcacacagattgtctgatgaagaacgagcaaaagcgcgtctgcgaagctgactgaagtcccttcgtctagaggcctaggacaccgcccttcacggcggtaacaggggttcgaatcccctaggg acgccaattgcgcggtatgagtgaaaggcgtaccacactatagtctgatgagaatcagagaatagttaagatattcgcatgagttatttacctattatgctcttaacaatctggaacaagctgaaaaattgaaaacaaatcaatatatcaccgagg tatattgatgagtctctcaaaatctcaaactttgaatgtgttttgacatcaaagtggatgagcgagcatttacagttcgagccggccagcgcgcagccagcgcaacatacataagtatgtgagcatggcgagcactgcccaacgacgaaatgt aatctgcgcagccatcaccaccaagatagtgtcttcaaaagacaccttcgggttgtg |
| 16 | cctaa[?][?][?][?][?][?][?][?][?][?]tatgtccagtgctcacacagattgtctgatgaagaacgagcaaaagcgcgtctgcgaagctgactgaagtcccctcgtctagaggcctaggacaccgcccttcacggcggtaacaggggttcgaatcccctagggg |
| | acgccaattgcgcggtatgagtgaaaggcgtaccacactatagtctgatgcaaatcagggaatagttaagataattcgatgagtttattttacctattatgctctttaacaatctggaacaagctgaaaaattgaaaacaaatcaatatatcaccgaggt atattgatgagtctctcaaaatctcaaatcttgaagttgtcactcaaagacgaatgtcatgagcgagcaacagcaattctaggcggacagcacgcagtaagcgcagcatacttaagtatgtgagcattacgagtactgcccaacaacgaattgatg cttgtgtagccatgaccttaaatatcctctttgaagaaacaccttcgggttgtga |
| 17 | cctaagagatacgtgttatgtgcagtgctcacacagatgtctgatgaagaacgagcaaaagcggtctgcgaagctgactgaagtcccttcgtctagaggcctaggacaccgccctttcacggcggtaacagggggttcgaatcccctagggg acgccaattgcgcggtatgagtgaaaggcgtaccacactatagtctgatgagaatcagagaatagttaagataattcgcatgagttatttacctattatgctctttaacaatctggaacaagctgaaaaattgaaaacaaatcaatatatcaccgagg tatattgatgagtctctcaaaatctcagaccttgaatgtgtgatactccaaggcgagtgtcatgagcgagcaacagcaattctaggcggacagcgcgcagtaagcgcagcatacataagtatgtgagcattacgagcactgcccaacaacgaatt gatgcttgtgtagcccatgacctttaaagtcgtcttcgagagaaacaccttcgggttgtga |
| 18 | agagaatagttaagataattttagcaagttattttaactattatgctctttaacaat |
| 19 | acagtcagcgcaacatacattagtatgagcatggcage |
| 20 | acgacgaaatgtaatctgcacagccatcaccacccagacgtcatyaagagaaacatcttcgggttgtga |
| 21 | ggcgtaccacttatctgacg |
| 22 | cgtaccacttatctgacg |
| 23 | gcgtaccacttatctgacg |
| 24 | cgtaccacttatctgac |
| 25 | cacacagattgtctgatgaagacgagcaaa |
| 26 | ctcacacagattgtctgatgaagaacgagcaaa |
| 27 | cgccaatgcgcggt |
| 28 | acgccaatgcgcggt |
| 29 | gacgccaatgcgcggt |
| 30 | tgaaaacaaatcaatatatcaccgaggtatat |
| 31 | attgaaaacaaatcaatatatcaccgaggtatat |
| 32 | tggaacaagctgaaaaattg |
| 33 | ggaacaagctgaaaaattg |
| 34 | gtgaattattatgctctttaacaatc |
| 35 | ttaaaggtactccctttaaaag |
| 36 | ggaacaactgaaaaattgaaaacaaatca |
| 37 | agtcagagaataactaagctaattca |
| 38 | agtcagagaataactaagctaattcaaa |
| 39 | gagtcagagaataactaagctaattca |
| 40 | gcgtctgcgaagctgac |
| 41 | cgcgtctgcgaagctg |
| 42 | tgagtgaaaggcgtace |
| 43 | tgagtctctcaaaatctcaaa |
| 44 | tgagtctctcaaaatctcaa |
| 45 | aacaaatcaatatatcaccgaggtatattgatga |
| 46 | aacaaatcaatatatcaccgaggtatattgat |
| 47 | ggaacaagctgaaaaattgaaaacaaatc |
| 48 | gtaagtaatcggtattaa |
| 49 | atatattaccgaggtatattgatgagt |
| 50 | gacgccaattgcgcggtatgagtgaa |
| 51 | ggcgtaccacttatctgac |
| 52 | ggcgtaccacactatagtctgat |
| 53 | cctaagagatacgtagttatgtg |
| 54 | taatctcttgratataaaacaatgattcagagtatattaggaatagtatactgygaattat |
| 55 | atataaaacaatgattcagagtatattaggaatagtatactg |
| 56 | taatctcttgaatataaaataataattcafaftatattagcaatagtatactgcgaattayttt |
| 57 | atataaaataataattcagagtatattagcaatagtatactg |
| 58 | tattwtgctctttaacaatctggaacaagctgaaaaattgaaaacaaatcaatatatcmcgaggtatattgrtgatctcaaaatctcara |
| 59 | tgctctttaacaatctggaacaagctgaaaaattgaaaacaaatcaatatatca |
| 60 | aaygagcagaaataccggtata |
| 61 | gtgctcacacagattgtctgatgaagaacgagca |
| 62 | agaacgagcaaaagcgcgtctgcgaagctgac |
| 63 | gacgccaatngcgcggtatgagtgaaaggcgtaccac |
| 64 | tgcgcggtatgagygaaaggcgtaccac |
| 65 | Ggcgtaccacttatctgacraragtcagagaataaytaagctaattcaaaygagttatcttayt |
| 66 | Ggcgtaccacttatctgacraragtcagagaataaytaagctaattcaaaygagttat |
| 67 | Ggcgtaccacttatctgacraragtcagagaataaytaagctaattcaaa |
| 68 | Acaccgcccgtcacaccayg |
| 69 | Astgccarggcatccacc |

## Claims

1. A set of at least two polynucleotide probes for the detection and/or identification of *Proteus* species, each probe comprising 5 to 50 nucleotides and said probes hybridizing specifically at adjacent positions separated by no more than 25 nucleotides between said two probes in any of nucleic acids selected from the group consisting of SEQ ID NO 1 to 17, their RNA form wherein T is replaced by U, the complementary form thereof, or equivalent homologous polynucleotides sharing at least 80% homology with the corresponding unmodified polynucleotides of SEQ ID NO 1 to 17.

2. A set of at least two polynucleotide probes according to claim 1, wherein said probes are selected from the group consisting of SEO ID NO 18 to 67.

3. A set of three polynucleotide probes according to claim 1 or 2, each probe comprising 5 to 50 nucleotides and said probes hybridizing specifically at adjacent positions separated by no more than 25 nucleotides between adjacent probes in any of nucleic acids selected from the group consisting of SEQ ID NO 1 to 17, their RNA form wherein T is replaced by U, the complementary form thereof, or equivalent homologous polynucleotides sharing at least 80% homology with the corresponding unmodified polynucleotides of SEQ ID NO 1 to 17.

4. A composition comprising at least one set of at least two polynucleotide probes described in any one of claims 1 to 3.

5. A method for detecting or identifying *Proteus* species using at least one set of at least two polynucleotide probes as set forth in any of the claims 1 to 3, wherein said probes are selected from the group consisting of SEO ID NO 18 to 67.

6. A method according to claim 5 for detection and/or identification of *Proteus* species in a sample comprising the steps of:
(i) releasing, isolating and/or concentrating the polynucleic acids in the sample;
(ii) amplifying the 16S-23S rRNA spacer region(s), or at least one of the target sequence(s) which comprise(s) any nucleic acid molecule(s) as described in claim 1, with at least one suitable primer pair;
(iii) contacting the polynucleic acids with at least one set of at least two polynucleotide probes hybridizing specifically at adjacent positions separated by no more than 25 nucleotides between adjacent probes, said probes selected from the group consisting of SEQ ID NO 18 to 67;
(iv) detecting the hybrids formed, and
(v) interpreting the signal(s) obtained and inferring the presence of *Proteus* species and/or identifying the *Proteus* species in the sample.

7. A method according to claim 5 or 6 wherein the two polynucleotide probes consist of any combination of polynucleotides of Table 2.

8. A kit for detection and/or identification of *Proteus* species comprising the following components:
- a set of at least two polynucleotide probes according to any one of claims 1 to 3,
- a hybridization buffer, or components necessary for producing said buffer.

## Patentansprüche

1. Satz von wenigstens zwei Polynukleotidsonden zum Nachweis und/oder zur Identifizierung von *Proteus-*Spezies, wobei die Sonden jeweils 5 bis 50 Nukleotide umfassen und spezifisch an benachbarte Positionen, die durch nicht mehr als 25 Nukleotide zwischen den zwei Sonden getrennt sind, in einer der Nukleinsäuren hybridisieren, die aus der aus SEQ ID NO: 1 bis 17, deren RNA-Form, wobei T durch U ersetzt ist, der komplementären Form davon oder äquivalenten homologen Polynukleotiden, die wenigstens 80% Homologie mit den entsprechenden unmodifizierten Polynukleotiden unter SEQ ID NO: 1 bis 17 teilen, bestehenden Gruppe ausgewählt sind.

2. Satz von wenigstens zwei Polynukleotidsonden nach Anspruch 1, wobei die Sonden aus der aus SEQ ID NO: 18 bis 67 bestehenden Gruppe ausgewählt sind.

3. Satz von drei Polynukleotidsonden nach Anspruch 1 oder 2, wobei die Sonden jeweils 5 bis 50 Nukleotide umfassen und spezifisch an benachbarte Positionen, die durch nicht mehr als 25 Nukleotide zwischen benachbarten Sonden getrennt sind, in einer der Nukleinsäuren hybridisieren, die aus der aus SEQ ID NO: 1 bis 17, deren RNA-Form, wobei T durch U ersetzt ist, der komplementären Form davon oder äquivalenten homologen Polynukleotiden, die wenigstens 80% Homologie mit den entsprechenden unmodifizierten Polynukleotiden unter SEQ ID NO: 1 bis 17 teilen, bestehenden Gruppe ausgewählt sind.

4. Zusammensetzung, umfassend wenigstens einen Satz von wenigstens zwei Polynukleotidsonden beschrieben in einem der Ansprüche 1 bis 3.

5. Verfahren zum Nachweisen oder Identifizieren von Proteus-Spezies unter Verwendung wenigstens eines Satzes von wenigstens zwei Polynukleotidsonden gemäß einem der Ansprüche 1 bis 3, wobei die Sonden aus der aus SEQ ID NO: 18 bis 67 bestehenden Gruppe ausgewählt sind.

6. Verfahren nach Anspruch 5 zum Nachweis und/oder zur Identifizierung von Proteus-Spezies in einer Probe, umfassend die Schritte:
(i) Freisetzen, Isolieren und/oder Konzentrieren der Polynukleinsäuren in der Probe;
(ii) Amplifizieren der 16S-23S-rRNA-Spacer-Region(en) oder wenigstens einer der Zielsequenz(en), die ein Nukleinsäuremolekül bzw. Nukleinsäuremoleküle wie in Anspruch 1 beschrieben umfasst bzw. umfassen, mit wenigstens einem geeigneten Primer-Paar;
(iii) Inkontaktbringen der Polynukleinsäuren mit wenigstens einem Satz von wenigstens zwei Polynukleotidsonden, die spezifisch an benachbarte Positionen, die durch nicht mehr als 25 Nukleotide zwischen benachbarten Sonden getrennt sind, hybridisieren, wobei die Sonden aus der aus SEQ ID NO: 18 bis 67 bestehenden Gruppe ausgewählt sind;
(iv) Nachweisen der gebildeten Hybride und
(v) Interpretieren des erhaltenen Signals bzw. der erhaltenen Signale und Schließen auf das Vorliegen von Proteus-Spezies und/oder Identifizieren der Proteus-Spezies in der Probe.

7. Verfahren nach Anspruch 5 oder 6, wobei die beiden Polynukleotidsonden aus einer beliebigen Kombination von Polynukleotiden in Tabelle 2 bestehen.

8. Kit zum Nachweis und/oder zur Identifizierung von Proteus-Spezies, umfassend die folgenden Komponenten:
- einen Satz von wenigstens zwei Polynukleotidsonden nach einem der Ansprüche 1 bis 3,
- einen Hybridisierungspuffer oder zur Herstellung des Puffers notwendige Komponenten.

## Revendications

1. Ensemble d'au moins deux sondes polynucléotidiques pour la détection et/ou l'identification d'une espèce de *Proteus,* chaque sonde comprenant 5 à 50 nucléotides, et lesdites sondes s'hybridant d'une manière spécifique sur des positions adjacentes séparées d'au plus 25 nucléotides entre lesdites deux sondes dans l'un quelconque des acides nucléiques choisis dans le groupe consistant en SEQ ID NO:1 à 17, leur forme ARN dans laquelle T est remplacé par U, sa forme complémentaire ou des polynucléotides homologues équivalents partageant une homologie d'au moins 80 % avec les polynucléotides non modifiés correspondants SEQ ID NO:1 à 17.

2. Ensemble d'au moins deux sondes polynucléotidiques selon la revendication 1, lesdites sondes étant choisies dans le groupe consistant en SEQ ID NO:18 à 67.

3. Ensemble de trois sondes polynucléotidiques selon la revendication 1 ou 2, chaque sonde comprenant 5 à 50 nucléotides, et lesdites sondes s'hybridant spécifiquement sur des positions adjacentes séparées de pas plus de 25 nucléotides entre des sondes adjacentes dans l'un quelconque des acides nucléiques choisis dans le groupe consistant en SEQ ID NO:1 à 17, leur forme ARN dans laquelle T est remplacé par U, sa forme complémentaire, ou des polynucléotides homologues équivalents partageant une homologie d'au moins 80 % avec les polynucléotides non modifiés correspondants SEQ ID NO:1 à 17.

4. Composition comprenant au moins un ensemble d'au moins deux sondes polynucléotidiques décrit dans l'une quelconque des revendications 1 à 3.

5. Procédé pour la détection ou l'identification d'une espèce de *Proteus* par utilisation d'au moins un ensemble d'au moins deux sondes polynucléotidiques tel que présenté dans l'une quelconque des revendications 1 à 3, dans lequel lesdites sondes sont choisies dans le groupe consistant en SEQ ID NO:18 à 67.

6. Procédé selon la revendication 5 pour la détection et/ou l'identification d'une espèce de *Proteus* dans un échantillon, comprenant les étapes de :
(i) libération, isolement et/ou concentration des poly(acides nucléiques) dans l'échantillon ;
(ii) amplification de la ou des régions intergéniques de l'ARNr 16S-23S, ou d'au moins l'une de la ou des séquences cibles qui comprennent une ou plusieurs molécules d'acides nucléiques quelconques telles que décrites dans la revendication 1, avec au moins une paire d'amorces appropriées ;
(iii) Mise en contact des poly(acides nucléiques) avec au moins un ensemble d'au moins deux sondes polynucléotidiques s'hybridant d'une manière spécifique sur des positions adjacentes séparées par pas plus de 25 nucléotides entre des sondes adjacentes, lesdites sondes étant choisies dans le groupe consistant en SEQ ID NO:18 à 67 ;
(iv) détection des hybrides formés, et
(v) interprétation du ou des signaux obtenus, et déduction de la présence d'une espèce de *Proteus* et/ou identification de l'espèce de *Proteus* dans l'échantillon.

7. Procédé selon la revendication 5 ou 6, dans lequel les deux sondes polynucléotidiques consistent en une combinaison quelconque des polynucléotides du Tableau 2.

8. Trousse pour la détection et/ou l'identification d'une espèce de *Proteus,* comprenant les composants suivants :
- un ensemble d'au moins deux sondes polynucléotidiques selon l'une quelconque des revendications 1 à 3,
- un tampon d'hybridation, ou les composants nécessaires à la production dudit tampon.
